# EUROPÄISCHE PATENTANMELDUNG

(11) **EP 1 142 869 A2**
(43) Veröffentlichungstag der Anmeldung: **10.10.2001**
(21) Anmeldenummer: 01107414.3
(22) Anmeldetag: 27.03.2001
(51) Int. Cl.: C07C 333/16

(54) **Verfahren zur Herstellung von Zink-Dithiocarbamaten**

(30) Priorität: 06.04.2000 DE 10017103
(71) Anmelder: BAYER AG, 51368 Leverkusen (DE)
(72) Erfinder: Schubart, Rüdiger, Dr., 51467 Bergisch Gladbach (DE); Engels, Hans-Wilhelm, Dr., 50170 Kerpen (DE)

(57) **Zusammenfassung**

Zink-Dithiocarbamate werden in besonders gut filtrierbarer Form und großer Reinheit erhalten, wenn man die den Zink-Carbamaten zugrundeliegenden sekundären Amine zunächst in wässriger Lösung mit Zinksalzen versetzt, so dass ein Zinksalzkomplex der Amine ausfällt, anschließend in die so erhaltene wässrige Suspension Schwefelkohlenstoff und anschließend Natronlauge unter Rühren einträgt, wobei die Temperaturen im Bereich von Raumtemperatur bis Siedetemperatur von Schwefelkohlenstoff so lange gehalten werden, bis die Umsetzung vollständig abgelaufen ist.

## Beschreibung

Die Erfindung betrifft ein Verfahren zur Herstellung von Zink-Dithiocarbamaten, wobei die Zink-Dithiocarbamate in besonders gut filtrierbarer Form und großer Reinheit anfallen.

Die Herstellung von Zink-Dithiocarbamaten, die vor allem im Pflanzenschutz als Fungizide und in der Gummiindustrie als Vulkanisationsbeschleuniger für natürliche und synthetische Kautschuke eingesetzt werden, erfolgt in üblicher Weise durch Umsetzung von sekundären Aminen mit Schwefelkohlenstoff in Gegenwart von Ammoniak oder Alkalilauge zu den entsprechenden Dithiocarbamaten, die anschließend mit Zinksalzen in die schwerlöslichen Zink-Dithiocarbamate überführt werden.

Eine weitere Möglichkeit zur Herstellung von Zink-Dithiocarbamaten ist die Umsetzung von sekundären Aminen in Gegenwart von Zinkoxid und Schwefelkohlenstoff.

Nachteilig bei den bekannten Verfahren zur Herstellung von Zink-Dithiocarbamaten ist vor allem, dass die Zink-Dithiocarbamate in kleiner Korngröße und unregelmäßiger Korngrößenverteilung anfallen, und daher schwer filtrierbar sind. Da die erhaltenen Korngrößen stark von der jeweiligen Fällungstemperatur der Zink-Dithiocarbamate aus dem Reaktionsgemisch abhängen - größere Korngröße bei höherer Fällungstemperatur -, wäre eine Möglichkeit um zu größeren Korngrößen zu gelangen, die Fällungstemperatur zu erhöhen. Diese Möglichkeit wird aber dadurch eingeschränkt, dass der eingesetzte Schwefelkohlenstoff einen niedrigen Siedepunkt hat und damit leicht flüchtig ist. Des weiteren ist Schwefelkohlenstoff leicht brennbar. Aus diesem Grund ist die Fällung der Zink-Dithiocarbamate bei höherer Temperatur problematisch.

Aufgabe der vorliegenden Erfindung ist es nun, ein einfaches Verfahren zur Herstellung von Zink-Dithiocarbamaten zur Verfügung zu stellen, das insbesondere die Zink-Dithiocarbamate in gut filtrierbarer Form liefert, d.h. mit großer Korngröße und einer möglichst gleichmäßigen Verteilung der erhaltenen Korngrößen.

Gegenstand der Erfindung ist daher ein Verfahren zur Herstellung von Zink-Dithiocarbamaten der Formel 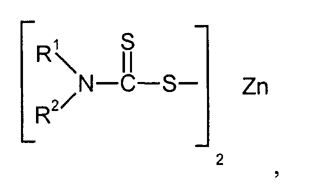 worin
- R¹ und R²: gleich oder verschieden, bevorzugt gleich sind, und für C₁-C₆-Alkyl- oder C₇-C₁₂-Aralkylreste stehen oder worin R¹ und R² durch Polymethylenketten mit 2 bis 12 Methylensegmente verbrückt sind, wobei die Polymethylenketten durch 1 bis 3 Heteroatome, wie Sauerstoff, Schwefel oder Stickstoff, unterbrochen sein können,
das dadurch gekennzeichnet ist, dass man die den Zink-Carbamaten zugrundeliegenden sekundären Amine zunächst in wässriger Lösung mit Zinksalzen versetzt, so dass ein Zinksalzkomplex der Amine ausfällt, anschließend in die so erhaltene wässrige Suspension Schwefelkohlenstoff und Natronlauge unter Rühren einträgt, wobei die Temperaturen bei Raumtemperatur (ca. 20°C) bis zur Siedetemperatur von Schwefelkohlenstoff (42°C bei Normaldruck) so lange gehalten werden, bis die Umsetzung vollständig abgelaufen ist.

Das erfindungsgemäße Verfahren kann am Beispiel der Herstellung von Zink-Dibenzyldithiocarbamat durch folgendes Formelschema wiedergegeben werden:

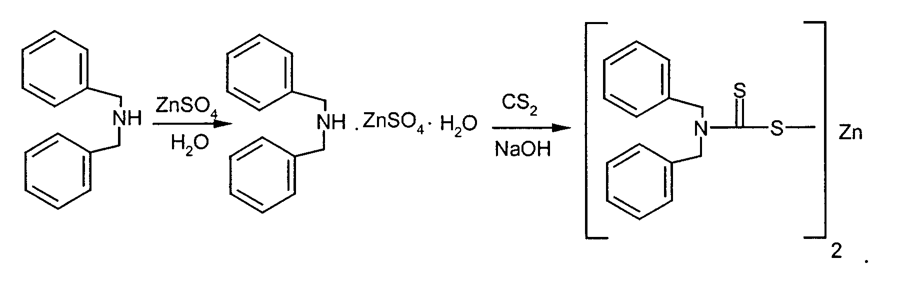

Als sekundäre Amine, die zur Herstellung der Dithiocarbamate eingesetzt werden können, kommen beispielsweise in Frage: Dimethylamin, Diethylamin, Dipropylamin, Dibutylamin, Piperidin, Morpholin, N-Methylpiperazin, N-Ethylpiperazin, Dibenzylamin, Diarylamin, Ethylanilin, Methylanilin, Methyl-tert.-butylamin sowie Ethyl-tert.-butylamin.

Bevorzugt sind Dibenzylamin, Dimethylamin, Diethylamin, Dibutylamin, Piperidin und Morpholin, ganz besonders bevorzugt ist Dibenzylamin.

Als Zinksalze, die erfindungsgemäß mit den sekundären Aminen in wässriger Lösung, bevorzugt unter Inertgasatmosphäre (z.B. Stickstoff) umgesetzt werden, kommen z.B. in Betracht: Zinksulfat, Zinkchlorid und/oder Zinkacetat, insbesondere Zinksulfat.

Die Zinksalze werden bevorzugt in 1 bis 25 %iger wässriger Lösung mit den sekundären Aminen umgesetzt, wobei die Zinksalze zu den sekundären Aminen im Verhältnis 0,8 bis 1,2:1, bevorzugt 1:1, eingesetzt werden.

Die dabei erhaltenen Zinksalzkomplexe der sekundären Amine werden anschließend mit Schwefelkohlenstoff umgesetzt, wobei die Menge an Schwefelkohlenstoff in einem leichten Überschuss bis maximal 5 Gew.-%, bezogen auf die Menge an Zinksalzkomplexe der sekundären Amine, verwendet werden kann.

Die Umsetzung der Zinksalzkomplexe der sekundären Amine mit Schwefelkohlenstoff geschieht dabei unter kräftigem Rühren, wobei die Suspension bevorzugt bei 30 bis 40°C gehalten wird.

Unter Zufügung von Alkalilauge, bevorzugt Natronlauge (5 bis 10%ig), wird solange gerührt, bei der angegebenen Temperatur, bis die Umsetzung vollständig abgelaufen ist. Die Natronlauge wird normalerweise stöchiometrisch eingesetzt. Ein leichter Überschuss von bis etwa 5 Gew.-% ist möglich.

Das ausgefallene Zink-Dithiocarbamat wird anschließend filtriert, gewaschen, beispielsweise mit Wasser und/oder mit Isopropanol, und dann getrocknet, vorteilhafterweise im Vakuum.

Die Zink-Dithiocarbamate werden nach dem erfindungsgemäßen Verfahren mit durchschnittlichen Primärkorngrößen erhalten, die das mehrfache der Normalgröße betragen, wobei die Korngrößenverteilung recht homogen ist. Im Falle des Zink-Dibenzyldithiocarbamates fällt das Primärkorn statt wie nach der bisherigen Syntheseweise mit 5 µ, erfindungsgemäß jetzt mit etwa 100 µ an, wie anhand der REM-Aufnahme zu ersehen ist. Die Ausbeuten nach dem erfindungsgemäßen Verfahren können über 99 % der Theorie erreichen. In der Regel liegen sie bei 98 bis 99 %.

Bei dem erfindungsgemäßen Verfahren hat es sich besonders bei längerkettigen Aminen als vorteilhaft erwiesen, wenn die Umsetzung der sekundären Amine in wässriger Lösung mit den Zinksalzen in Gegenwart eines Emulgators, wie Emulgator® L3, der Bayer AG, durchgeführt wird. Die Menge an Emulgator beträgt dabei etwa 0,1 bis 0,5 Gew.-%, bezogen auf eingesetztes sekundäres Amin, bevorzugt jedoch 0,2 bis 0,3 Gew.-%.

### Beispiele

### Beispiel 1

### Herstellung von Zink-Dibenzyldithiocarbamat

### Herstellung

Zu 788 g Dibenzylamin, 2 g Emulgator® L3 (Bayer AG) und 800 g Wasser wird unter Stickstoff innerhalb von 16 Minuten eine Lösung aus 575 g Zinksulfat (mit 7 Mol Kristallwasser) und 3 200 g Wasser bei 20 bis 21°C unter gutem Rühren (10 l Kolben) eingetropft.

Es fällt ein weißer, grobkristalliner Niederschlag aus, der nach der Analyse ein Dibenzylaminaddukt an Zinksulfat mit 3 Mol Kristallwasser darstellt. In diese Suspension werden 314 g Schwefelkohlenstoff durch einen mit einem verlängerten Auslauf versehenen Tropfrichter innerhalb einer Stunde unter die Oberfläche der Suspension getropft. Die Temperatur steigt dabei von 21 auf 24°C an. Jetzt wird innerhalb von 38 Minuten bis 35°C erwärmt. Man tropft nun unter weiterem Erwärmen eine Lösung von 160,2 g Natriumhydroxid und 2 000 g Wasser innerhalb von 40 Minuten zu, wobei die Temperatur bis 40°C ansteigt. Es wird 70 Minuten bei 40°C gerührt, dann warm abgesaugt und mehrmals mit 80°C heißem Wasser gewaschen. Das Produkt lässt sich sehr gut, innerhalb weniger Minuten absaugen. Anschließend wird im Vakuum bei 50°C über Natriumhydroxid getrocknet. Man erhält 1 203 g eines fast weißen Zink-Dibenzyldithiocarbamates vom Schmelzpunkt 185 bis 187°C. Die Ausbeute beträgt 98,9 % d.Th.

Die Korngröße der Kristallite beträgt nach REM-Aufnahme durchschnittlich etwa 100 µ.

### Beispiel 2 (Vergleich)

788 g Dibenzylamin werden bei 10°C in 2 l Wasser und 160 g Natronlauge gerührt. Innerhalb von 2,5 Stunden gibt man 304,5 g Schwefelkohlenstoff hinzu. Dabei erwärmt sich die Mischung innerhalb 30 min auf 30°C.

Bei dieser Temperatur lässt man nach der Zugabe des Schwefelkohlenstoffs noch 4 Stunden rühren.

Anschließend gibt man 3 l Wasser hinzu und fügt bei 40°C innerhalb 2 Stunden 273,5 g ZnCl₂ (gelöst in 1 l Wasser) zu und lässt 3 bis 4 Stunden nachrühren.

Das Absaugen der breiigen Mischung benötigt mehrere Stunden.

Die Primärkorngröße des erhaltenen Carbamats beträgt ca. 2 bis 3 µ im Durchschnitt.

## Patentansprüche

1. Verfahren zur Herstellung von Zink-Dithiocarbamaten der Formel 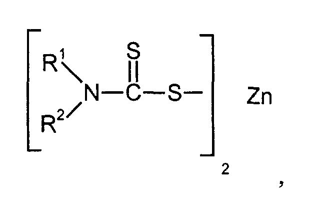 worin
R¹ und R² gleich oder verschieden sind und für einen C₁-C₆-Alkyl- oder C₇-C₁₂-Aralkylrest stehen
oder worin
R¹ und R² durch Polymethylenketten mit 2 bis 12 Methylensegmenten verbrückt, wobei die Polymethylenketten durch 1 bis 3 Heteroatome unterbrochen sein können,
**dadurch gekennzeichnet, dass** man die den Zink-Carbamaten zugrundeliegenden sekundären Amine zunächst in wässriger Lösung mit Zinksalzen versetzt, so **dass** ein Zinksalzkomplex der Amine ausfällt, anschließend in die so erhaltene wässrige Suspension Schwefelkohlenstoff und anschließend Natronlauge unter Rühren einträgt, wobei die Temperaturen im Bereich von Raumtemperatur bis Siedetemperatur von Schwefelkohlenstoff so lange gehalten werden, bis die Umsetzung vollständig abgelaufen ist.

2. Verfahren nach Anspruch 1, **dadurch gekennzeichnet, dass** das Molverhältnis von eingesetzten Zinksalzen zu eingesetzten sekundären Aminen 0,8 bis 1,2:1 beträgt.

3. Verfahren nach Anspruch 1, **dadurch gekennzeichnet, dass** der eingehaltene Temperaturbereich 30 bis 40°C beträgt.
